# FASCICULE DE BREVET EUROPEEN

(11) **EP 2 379 176 B1**
(45) Date de publication et mention de la délivrance du brevet: **14.02.2018**
(21) Numéro de dépôt: 09805752.4
(22) Date de dépôt: 18.12.2009
(51) Int. Cl.: A61K 8/31, A61K 8/41, A61K 8/22, A61K 8/86, A61K 8/06, A61Q 5/08

(54) **PROCEDE D'ECLAIRCISSEMENT DES CHEVEUX METTANT EN OEUVRE UNE EMULSION DIRECTE COMPRENANT UN AGENT OXYDANT ET UNE COMPOSITION CONTENANT UN AGENT ALCALIN**
VERFAHREN ZUR AUFHELLUNG VON HAAREN BEI VORHANDENSEIN EINER OXIDATIONSMITTEL ENTHALTENDEN DIREKTEN EMULSION UND EINEM ALKALISIERUNGSMITTEL
METHOD FOR LIGHTENING HAIR USING A DIRECT EMULSION COMPRISING AN OXIDISING AGENT AND AN ALKALINE AGENT

(30) Priorité: 06.02.2009 US 150437 P; 19.12.2008 FR 0807323
(43) Date de publication de la demande: 26.10.2011
(73) Titulaire: L'Oréal, 75008 Paris (FR)
(72) Inventeur: HERCOUET, Leila, 93360 Neuilly Plaisance (FR); SIMONET, Frédéric, 92110 Clichy (FR); BERNARD, Anne-Laure, 92160 Antony (FR)
(74) Mandataire: Fevrier, Murielle Françoise E.
(86) Numéro de dépôt international: PCT/FR2009/052617
(87) Numéro de publication internationale: WO 2010/070243

(56) Documents cités:
- EP-A1- 1 716 839
- DE-C1- 19 723 538
- FR-A- 2 616 324
- JP-A- 2003 055 174
- US-A- 4 170 637
- US-A1- 2003 190 297
- US-A1- 2006 075 580

## Description

La présente invention a pour objet un procédé de traitement des matières kératiniques humaines, notamment d'éclaircissement et/ou de coloration des cheveux à partir d'une émulsion directe particulière.

En cosmétique, dans les domaines de la teinture et de la décoloration des fibres kératiniques, et en particulier des fibres kératiniques humaines telles que les cheveux, on utilise des compositions oxydantes.

Ainsi, en teinture d'oxydation des cheveux, des compositions oxydantes sont mélangées aux colorants d'oxydation (bases et coupleurs), qui sont incolores par eux-mêmes, pour engendrer des composés colorés et colorants par un processus de condensation oxydative. Des compositions oxydantes sont également utilisées en teinture directe des cheveux en mélange avec certains colorants directs qui sont colorés et colorants pour obtenir une coloration avec un effet éclaircissant des cheveux. Parmi les agents oxydants classiquement utilisés pour la teinture des fibres kératiniques, on peut citer le peroxyde d'hydrogène ou des composés susceptibles de produire le peroxyde d'hydrogène par hydrolyse tels que le peroxyde d'urée, les persels comme les perborates et persulfates, le peroxyde d'hydrogène étant plus particulièrement préféré.

En décoloration des cheveux, les compositions de décoloration contiennent un ou plusieurs agents oxydants. Parmi ces agents oxydants, les plus classiquement utilisés sont le peroxyde d'hydrogène ou des composés susceptibles de produire le peroxyde d'hydrogène par hydrolyse, tels que le peroxyde d'urée. On peut aussi utiliser des persels comme les perborates, les percarbonates et les persulfates

Ces compositions peuvent être des compositions aqueuses contenant des agents alcalins (amines ou ammoniaque) que l'on mélange au moment de l'emploi avec une composition aqueuse de peroxyde d'hydrogène.

Ces compositions peuvent aussi être formées de produits anhydres qui contiennent des composés alcalins (amines et silicates alcalins), et un réactif peroxygéné tels que les persulfates, perborates ou percarbonates, d'ammonium ou de métaux alcalins, que l'on dilue au moment de l'emploi avec une composition aqueuse de peroxyde d'hydrogène.

L'introduction d'une teneur importante en huile, en remplacement de l'eau, dans les compositions de coloration et / ou de décoloration des fibres kératiniques permet d'améliorer les performances des actifs éclaircissants. Cependant, l'introduction d'une teneur importante en huile dans la composition oxydante conduit à une déstabilisation de la composition qui déphase au bout de quelques jours.

Le but de la présente invention est de fournir de nouvelles compositions oxydantes qui permettent d'améliorer les propriétés éclaircissantes des compositions de coloration et / ou de décoloration et qui sont stables dans le temps, tout en restant au moins aussi efficaces sur le plan de l'éclaircissement et de l'homogénéité de ce dernier et en préservant la qualité de la fibre kératinique.

Ces buts et d'autres sont atteints par la présente invention qui a donc pour objet un procédé d'éclaircissement de matières kératiniques qui consiste à traiter les matières kératiniques avec au moins a) une émulsion directe (A) huile dans eau comprenant un ou plusieurs corps gras différents des acides gras dont au moins une huile en quantité supérieure à 25 % en poids; un ou plusieurs tensioactifs non ioniques oxyéthylénés (OE) choisis parmi les alcools oxyéthylénés en C₁₈-C₃₀, le nombre de motifs OE étant compris entre 1 et 50 ; une quantité d'eau supérieure à 5 % en poids, du poids total de l'émulsion, et un ou plusieurs agents oxydants, et b) une composition comprenant un ou plusieurs agents alcalins choisis parmi les amines organiques.

Elle concerne également un dispositif à plusieurs compartiments comprenant dans l'un d'eux l'émulsion directe (A), dans un autre une composition (B) comprenant un ou plusieurs agents alcalins.

Dans le cadre de l'invention, une émulsion directe est une émulsion huile dans eau.

Dans ce qui va suivre, et à moins d'une autre indication, les bornes d'un domaine de valeurs sont comprises dans ce domaine.

Les matières kératiniques traitées par le procédé selon l'invention sont notamment les cheveux. Le procédé de l'invention permet en particulier d'obtenir un bon niveau d'éclaircissement des cheveux sans dégagement d'une odeur d'ammoniaque, pouvant être irritante et tout en préservant une bonne qualité de la fibre kératiniques.

L'émulsion (A) présente plus particulièrement une teneur en eau inférieure à 50 % en poids, de préférence comprise entre 10 et 50 % en poids, par rapport au poids de l'émulsion.

L'émulsion huile dans eau utile dans la présente invention comprend un ou plusieurs corps gras.

Par corps gras, on entend, un composé organique insoluble dans l'eau à température ordinaire (25°C) et à pression atmosphérique (760 mm de Hg) (solubilité inférieure à 5% et de préférence à 1% encore plus préférentiellement à 0,1%). Ils présentent dans leur structure au moins un enchaînement d'au moins deux groupements siloxane ou au moins une chaine hydrocarbonée comportant au moins 6 atomes de carbone. En outre, les corps gras sont généralement solubles dans les solvants organiques dans les mêmes conditions de température et de pression, comme par exemple le chloroforme, l'éthanol, le benzène, l'huile de vaseline, ou le décaméthylcyclopentasiloxane.

Les corps gras sont notamment choisis parmi les alcanes inférieurs, les alcools gras, les esters d'acide gras, les esters d'alcool gras, les huiles en particulier les huiles non siliconées minérales, végétales, animales ou synthétiques, les cires non siliconées et les silicones.

Il est rappelé qu'au sens de l'invention, les alcools, esters et acides gras présentent plus particulièrement un ou plusieurs groupements hydrocarbonés, linéaires ou ramifiés, saturés ou insaturés, comprenant 6 à 30 atomes de carbone, éventuellement substitués, en particulier par un ou plusieurs groupements hydroxyle (en particulier 1 à 4). S'ils sont insaturés, ces composés peuvent comprendre une à trois double-liaisons carbone-carbone, conjuguées ou non.

En ce qui concerne les alcanes inférieurs, ces derniers comprennent de préférence de 6 à 16 atomes de carbone et sont linéaires ou ramifiés, éventuellement cycliques. A titre d'exemple, les alcanes peuvent être choisis parmi l'hexane et le dodécane, les isoparaffines comme l'isohexadécane et l'isodécane.

Comme huiles non siliconées utilisables dans la composition de l'invention, on peut citer par exemple :
- les huiles hydrocarbonées d'origine animale, telles que le perhydrosqualène ;
- les huiles hydrocarbonées d'origine végétale, telles que les triglycérides liquides d'acides gras comportant de 6 à 30 atomes de carbone comme les triglycérides des acides heptanoïque ou octanoïque ou encore, par exemple les huiles de tournesol, de maïs, de soja, de courge, de pépins de raisin, de sésame, de noisette, d'abricot, de macadamia, d'arara, de tournesol, de ricin, d'avocat, les triglycérides des acides caprylique/caprique comme ceux vendus par la société Stearineries Dubois ou ceux vendus sous les dénominations Miglyol® 810, 812 et 818 par la société Dynamit Nobel, l'huile de jojoba, l'huile de beurre de karité ;
- les hydrocarbures linéaires ou ramifiés ayant plus de 16 atomes de carbone, d'origine minérale ou synthétique, tels que les huiles de paraffine, la vaseline, l'huile de vaseline, les polydécènes, le polyisobutène hydrogéné tel que Parléam®.
- les huiles fluorées partiellement hydrocarbonées ; comme huiles fluorées, on peut citer aussi le perfluorométhylcyclopentane et le perfluoro-1,3 diméthylcyclohexane, vendus sous les dénominations de "FLUTEC® PC1" et "FLUTEC® PC3" par la Société BNFL Fluorochemicals ; le perfluoro-1,2-diméthylcyclobutane ; les perfluoroalcanes tels que le dodécafluoropentane et le tétradécafluorohexane, vendus sous les dénominations de "PF 5050®" et "PF 5060®" par la Société 3M, ou encore le bromoperfluorooctyle vendu sous la dénomination "FORALKYL®" par la Société Atochem ; le nonafluoro-méthoxybutane et le nonafluoroéthoxyisobutane ; les dérivés de perfluoromorpholine, tels que la 4-trifluorométhyl perfluoromorpholine vendue sous la dénomination "PF 5052®" par la Société 3M.

Les alcools gras utilisables dans la composition de l'invention sont non oxyalkylénés, saturés ou insaturés, linéaires ou ramifiés, et comportent 6 à 30 atomes de carbone et plus particulièrement de 8 à 30 atomes de carbone, on peut citer l'alcool cétylique, l'alcool stéarylique et leur mélange (alcool cétylstéarylique), l'octyldodécanol, le 2-butyloctanol, le 2-hexyldécanol, le 2-undécylpentadécanol, l'alcool oléique ou l'alcool linoléique.

La cire ou les cires non siliconées susceptibles d'être utilisées dans la composition de l'invention sont choisies notamment, parmi la cire de Carnauba, la cire de Candelila, et la cire d'Alfa, la cire de paraffine, l'ozokérite, les cires végétales comme la cire d'olivier, la cire de riz, la cire de jojoba hydrogénée ou les cires absolues de fleurs telles que la cire essentielle de fleur de cassis vendue par la société BERTIN (France), les cires animales comme les cires d'abeilles, ou les cires d'abeilles modifiées (cerabellina) ; d'autres cires ou matières premières cireuses utilisables selon l'invention sont notamment les cires marines telles que celle vendue par la Société SOPHIM sous la référence M82, les cires de polyéthylène ou de polyoléfines en général.

Les esters sont les esters de mono ou polyacides aliphatiques saturés ou insaturés, linéaires ou ramifiés en C1-C26 et de mono ou polyalcools aliphatiques saturés ou insaturés, linéaires ou ramifiés en C1-C26, le nombre total de carbone des esters étant plus particulièrement supérieur ou égal à 10.

Parmi les monoesters, on peut citer le béhénate de dihydroabiétyle ; le béhénate d'octyldodécyle ; le béhénate d'isocétyle ; le lactate de cétyle ; le lactate d'alkyle en C12-C15 ; le lactate d'isostéaryle ; le lactate de lauryle ; le lactate de linoléyle ; le lactate d'oléyle ; l'octanoate de (iso)stéaryle ; l'octanoate d'isocétyle ; l'octanoate d'octyle ; l'octanoate de cétyle ; l'oléate de décyle ; l'isostéarate d'isocétyle ; le laurate d'isocétyle ; le stéarate d'isocétyle ; l'octanoate d'isodécyle ; l'oléate d'isodécyle ; l'isononanoate d'isononyle ; le palmitate d'isostéaryle ; le ricinoléate de méthyle acétyle ; le stéarate de myristyle ; l'isononanoate d'octyle ; l'isononate de 2-éthylhexyle ; le palmitate d'octyle ; le pélargonate d'octyle ; le stéarate d'octyle ; l'érucate d'octyldodécyle ; l'érucate d'oléyle ; les palmitates d'éthyle et d'isopropyle, le palmitate d'éthyl-2-héxyle, le palmitate de 2-octyldécyle, les myristates d'alkyles tels que le myristate d'isopropyle, de butyle, de cétyle, de 2-octyldodécyle, de mirystyle, de stéaryle le stéarate d'hexyle, le stéarate de butyle, le stéarate d'isobutyle ; le malate de dioctyle, le laurate d'hexyle, le laurate de 2-hexyldécyle.

Toujours dans le cadre de cette variante, on peut également utiliser les esters d'acides di ou tricarboxyliques en C4-C22 et d'alcools en C1-C22 et les esters d'acides mono di ou tricarboxyliques et d'alcools di, tri, tétra ou pentahydroxy en C2-C26.

On peut notamment citer : le sébacate de diéthyle ; le sébacate de diisopropyle ; l'adipate de diisopropyle ; l'adipate de di n-propyle ; l'adipate de dioctyle ; l'adipate de diisostéaryle ; le maléate de dioctyle ; l'undecylénate de glycéryle ; le stéarate d'octyldodécyl stéaroyl ; le monoricinoléate de pentaérythrityle ; le tétraisononanoate de pentaérythrityle ; le tétrapélargonate de pentaérythrityle ; le tétraisostéarate de pentaérythrityle ; le tétraoctanoate de pentaérythrityle ; le dicaprylate de propylène glycol ; le dicaprate de propylène glycol, l'érucate de tridécyle ; le citrate de triisopropyle ; le citrate de triisotéaryle ; trilactate de glycéryle ; trioctanoate de glycéryle ; le citrate de trioctyldodécyle ; le citrate de trioléyle, le dioctanoate de propylène glycol ; le diheptanoate de néopentyl glycol ; le diisanonate de diéthylène glycol ; et les distéarates de polyéthylène glycol.

Parmi les esters cités ci-dessus, on préfère utiliser les palmitates d'éthyle, d'isopropyle, de myristyle, de cétyle, de stéaryle, le palmitate d'éthyl-2-héxyle, le palmitate de 2-octyldécyle, les myristates d'alkyles tels que le myristate d'isopropyle, de butyle, de cétyle, de 2-octyldodécyle, le stéarate d'hexyle, le stéarate de butyle, le stéarate d'isobutyle ; le malate de dioctyle, le laurate d'hexyle, le laurate de 2-hexyldécyle et l'isononanate d'isononyle, l'octanoate de cétyle.

La composition peut également comprendre, à titre d'ester gras, des esters et diesters de sucres d'acides gras en C6-C30, de préférence en C12-C22. Il est rappelé que l'on entend par « sucre », des composés hydrocarbonés oxygénés qui possèdent plusieurs fonctions alcool, avec ou sans fonction aldéhyde ou cétone, et qui comportent au moins 4 atomes de carbone. Ces sucres peuvent être des monosaccharides, des oligosaccharides ou des polysaccharides.

Comme sucres convenables, on peut citer par exemple le sucrose (ou saccharose), le glucose, le galactose, le ribose, le fucose, le maltose, le fructose, le mannose, l'arabinose, le xylose, le lactose, et leurs dérivés notamment alkylés, tels que les dérivés méthylés comme le méthylglucose.

Les esters de sucres et d'acides gras peuvent être choisis notamment dans le groupe comprenant les esters ou mélanges d'esters de sucres décrits auparavant et d'acides gras en C6-C30, de préférence en C12-C22, linéaires ou ramifiés, saturés ou insaturés. S'ils sont insaturés, ces composés peuvent comprendre une à trois double-liaisons carbone-carbone, conjuguées ou non.

Les esters selon cette variante peuvent être également choisis parmi les mono-, di-, tri- et tétra-esters, les polyesters et leurs mélanges.

Ces esters peuvent être par exemple des oléate, laurate, palmitate, myristate, béhénate, cocoate, stéarate, linoléate, linolénate, caprate, arachidonates, ou leurs mélanges comme notamment les esters mixtes oléo-palmitate, oléo-stéarate, palmito-stéarate.

Plus particulièrement, on utilise les mono- et di- esters et notamment les mono- ou di- oléate, stéarate, béhénate, oléopalmitate, linoléate, linolénate, oléostéarate, de saccharose, de glucose ou de méthylglucose.

On peut citer à titre d'exemple le produit vendu sous la dénomination Glucate® DO par la société Amerchol, qui est un dioléate de méthylglucose.

On peut aussi citer à titre d'exemples d'esters ou de mélanges d'esters de sucre d'acide gras :
- les produits vendus sous les dénominations F160, F140, F110, F90, F70, SL40 par la société Crodesta, désignant respectivement les palmito-stéarates de sucrose formés de 73 % de monoester et 27 % de di- et tri-ester, de 61 % de monoester et 39 % de di-, tri-, et tétra-ester, de 52 % de monoester et 48 % de di-, tri-, et tétra-ester, de 45 % de monoester et 55 % de di-, tri-, et tétra-ester, de 39 % de monoester et 61 % de di-, tri-, et tétra-ester, et le mono-laurate de sucrose;
- les produits vendus sous la dénomination Ryoto Sugar Esters par exemple référencés B370 et correspondant au béhénate de saccharose formé de 20 % de monoester et 80 % de di-triester-polyester;
- le mono-di-palmito-stéarate de sucrose commercialisé par la société Goldschmidt sous la dénomination Tegosoft® PSE.

Les silicones utilisables dans la composition de la présente invention, sont des silicones volatiles ou non volatiles, cycliques, linéaires ou ramifiées, modifiées ou non par des groupements organiques, ayant une viscosité de 5.10-6 à 2,5m2/s à 25°C et de préférence 1.10-5 à 1 m2/s.

Les silicones utilisables conformément à l'invention peuvent se présenter sous forme d'huiles, de cires, de résines ou de gommes.

De préférence, la silicone est choisie parmi les polydialkylsiloxanes, notamment les polydiméthylsiloxanes (PDMS), et les polysiloxanes organo-modifiés comportant au moins un groupement fonctionnel choisi parmi les groupements poly(oxyalkylène), les groupements aminés et les groupements alcoxy.

Les organopolysiloxanes sont définis plus en détail dans l'ouvrage de Walter NOLL "Chemistry and Technology of Silicones" (1968), Academie Press. Elles peuvent être volatiles ou non volatiles.

Lorsqu'elles sont volatiles, les silicones sont plus particulièrement choisies parmi celles possédant un point d'ébullition compris entre 60°C et 260°C, et plus particulièrement encore parmi:
les polydialkylsiloxanes cycliques comportant de 3 à 7, de préférence de 4 à 5 atomes de silicium. Il s'agit, par exemple, de l'octaméthylcyclotétrasiloxane commercialisé notamment sous le nom de VOLATILE SILICONE® 7207 par UNION CARBIDE ou SILBIONE® 70045 V2 par RHODIA, le décaméthylcyclopentasiloxane commercialisé sous le nom de VOLATILE SILICONE® 7158 par UNION CARBIDE, et SILBIONE® 70045 V5 par RHODIA, ainsi que leurs mélanges.

On peut également citer les cyclocopolymères du type diméthylsiloxanes/ méthylalkylsiloxane, tel que la SILICONE VOLATILE® FZ 3109 commercialisée par la société UNION CARBIDE, de formule :

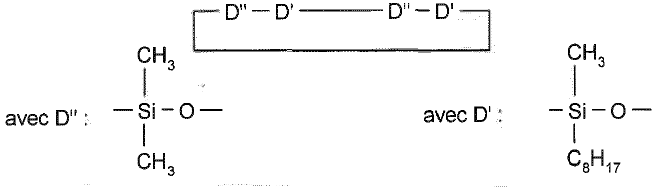

On peut également citer les mélanges de polydialkylsiloxanes cycliques avec des composés organiques dérivés du silicium, tels que le mélange d'octaméthylcyclotétrasiloxane et de tétratriméthylsilylpentaérythritol (50/50) et le mélange d'octaméthylcyclotétrasiloxane et d'oxy-1,1'-(hexa-2,2,2',2',3,3'-triméthylsilyloxy) bis-néopentane ;
(ii) les polydialkylsiloxanes volatiles linéaires ayant 2 à 9 atomes de silicium et présentant une viscosité inférieure ou égale à 5.10-6m²/s à 25° C. Il s'agit, par exemple, du décaméthyltétrasiloxane commercialisé notamment sous la dénomination "SH 200" par la société TORAY SILICONE. Des silicones entrant dans cette classe sont également décrites dans l'article publié dans Cosmetics and Toiletries, Vol. 91, Jan. 76, P. 27-32 - TODD & BYERS "Volatile Silicone fluids for cosmetics".

On utilise de préférence des polydialkylsiloxanes non volatiles, des gommes et des résines de polydialkylsiloxanes, des polyorganosiloxanes modifiés par les groupements organofonctionnels ci-dessus ainsi que leurs mélanges.

Ces silicones sont plus particulièrement choisies parmi les polydialkylsiloxanes parmi lesquels on peut citer principalement les polydiméthylsiloxanes à groupements terminaux triméthylsilyl. La viscosité des silicones est mesurée à 25°C selon la norme ASTM 445 Appendice C.

Parmi ces polydialkylsiloxanes, on peut citer à titre non limitatif les produits commerciaux suivants :
- les huiles SILBIONE® des séries 47 et 70 047 ou les huiles MIRASIL® commercialisées par RHODIA telles que, par exemple l'huile 70 047 V 500 000 ;
- les huiles de la série MIRASIL® commercialisées par la société RHODIA ;
- les huiles de la série 200 de la société DOW CORNING telles que la DC200 ayant viscosité 60 000 mm2/s ;
- les huiles VISCASIL® de GENERAL ELECTRIC et certaines huiles des séries SF (SF 96, SF 18) de GENERAL ELECTRIC.

On peut également citer les polydiméthylsiloxanes à groupements terminaux diméthylsilanol connus sous le nom de dimethiconol (CTFA), tels que les huiles de la série 48 de la société RHODIA.

Dans cette classe de polydialkylsiloxanes, on peut également citer les produits commercialisés sous les dénominations "ABIL WAX® 9800 et 9801" par la société GOLDSCHMIDT qui sont des polydialkyl (C1-C20) siloxanes.

Les gommes de silicone utilisables conformément à l'invention sont notamment des polydialkylsiloxanes, de préférence des polydiméthylsiloxanes ayant des masses moléculaires moyennes en nombre élevées comprises entre 200 000 et 1 000 000 utilisés seuls ou en mélange dans un solvant. Ce solvant peut être choisi parmi les silicones volatiles, les huiles polydiméthylsiloxanes (PDMS), les huiles polyphénylméthylsiloxanes (PPMS), les isoparaffines, les polyisobutylènes, le chlorure de méthylène, le pentane, le dodécane, le tridécane ou leurs mélanges.

Des produits plus particulièrement utilisables conformément à l'invention sont des mélanges tels que :
- les mélanges formés à partir d'un polydiméthylsiloxane hydroxylé en bout de chaîne, ou diméthiconol (CTFA) et d'un polydiméthylsiloxane cyclique également appelé cyclométhicone (CTFA) tel que le produit Q2 1401 commercialisé par la société DOW CORNING ;
- les mélanges d'une gomme polydiméthylsiloxane et d'une silicone cyclique tel que le produit SF 1214 Silicone Fluid de la société GENERAL ELECTRIC, ce produit est une gomme SF 30 correspondant à une diméthicone, ayant un poids moléculaire moyen en nombre de 500 000 solubilisée dans l'huile SF 1202 Silicone Fluid correspondant au décaméthylcyclopentasiloxane ;
- les mélanges de deux PDMS de viscosités différentes, et plus particulièrement d'une gomme PDMS et d'une huile PDMS, tels que le produit SF 1236 de la société GENERAL ELECTRIC. Le produit SF 1236 est le mélange d'une gomme SE 30 définie ci-dessus ayant une viscosité de 20 m²/s et d'une huile SF 96 d'une viscosité de 5.10-6m²/s. Ce produit comporte de préférence 15 % de gomme SE 30 et 85 % d'une huile SF 96.

Les résines d'organopolysiloxanes utilisables conformément à l'invention sont des systèmes siloxaniques réticulés renfermant les motifs :
R2SiO2/2 R3SiO1/2, RSiO3/2 et SiO4/2
dans lesquelles R représente un alkyl possédant 1 à 16 atomes de carbone. Parmi ces produits, ceux particulièrement préférés sont ceux dans lesquels R désigne un groupe alkyle inférieur en C1-C4, plus particulièrement méthyle.

On peut citer parmi ces résines le produit commercialisé sous la dénomination "DOW CORNING 593" ou ceux commercialisés sous les dénominations "SILICONE FLUID SS 4230 et SS 4267" par la société GENERAL ELECTRIC et qui sont des silicones de structure diméthyl/triméthyl siloxane.

On peut également citer les résines du type triméthylsiloxysilicate commercialisées notamment sous les dénominations X22-4914, X21-5034 et X21-5037 par la société SHIN-ETSU.

Les silicones organomodifiées utilisables conformément à l'invention sont des silicones telles que définies précédemment et comportant dans leur structure un ou plusieurs groupements organofonctionnels fixés par l'intermédiaire d'un groupe hydrocarboné.

Outre, les silicones décrites ci-dessus les silicones organomodifiées peuvent être des polydiaryl siloxanes, notamment des polydiphénylsiloxanes, et des polyalkylarylsiloxanes fonctionnalisés par les groupes organofonctionnels mentionnés précédemment.

Les polyalkylarylsiloxanes sont particulièrement choisis parmi les polydiméthyl/méthylphénylsiloxanes, les polydiméthyl/diphénylsiloxanes linéaires et/ou ramifiés de viscosité allant de 1.10-5 à 5.10-2m²/s à 25°C.

Parmi ces polyalkylarylsiloxanes, on peut citer à titre d'exemple les produits commercialisés sous les dénominations suivantes :
- les huiles SILBIONE® de la série 70 641 de RHODIA;
- les huiles des séries RHODORSIL® 70 633 et 763 de RHODIA ;
- l'huile DOW CORNING 556 COSMETIC GRAD FLUID de DOW CORNING ;
- les silicones de la série PK de BAYER comme le produit PK20 ;
- les silicones des séries PN, PH de BAYER comme les produits PN1000 et PH1000;
- certaines huiles des séries SF de GENERAL ELECTRIC telles que SF 1023, SF 1154, SF 1250, SF 1265.

Parmi les silicones organomodifiées, on peut citer les polyorganosiloxanes comportant :
- des groupements polyéthylèneoxy et/ou polypropylèneoxy comportant éventuellement des groupements alkyle en C6-C24 tels que les produits dénommés diméthicone copolyol commercialisé par la société DOW CORNING sous la dénomination DC 1248 ou les huiles SILWET® L 722, L 7500, L 77, L 711 de la société UNION CARBIDE et l'alkyl (C12)-méthicone copolyol commercialisée par la société DOW CORNING sous la dénomination Q2 5200 ;
- des groupements aminés substitués ou non comme les produits commercialisés sous la dénomination GP 4 Silicone Fluid et GP 7100 par la société GENESEE ou les produits commercialisés sous les dénominations Q2 8220 et DOW CORNING 929 ou 939 par la société DOW CORNING. Les groupements aminés substitués sont en particulier des groupements aminoalkyle en C1-C4 ;
- des groupements alcoxylés, comme le produit commercialisé sous la dénomination "SILICONE COPOLYMER F-755" par SWS SILICONES et ABIL WAX® 2428, 2434 et 2440 par la société GOLDSCHMIDT.

De préférence les corps gras sont non oxyalkylénés et non glycérolés.

Plus particulièrement, les corps gras sont choisis parmi les composés liquides ou pâteux à température ambiante et à pression atmosphérique.

De préférence, le corps gras comprend majoritairement un composé liquide à la température de 25°C et à la pression atmosphérique.

Les corps gras sont de préférence choisis parmi les alcanes inférieurs C6-C16, les alcools gras, les esters d'acide gras, les esters d'alcool gras, les huiles en particulier les huiles non siliconées minérales à plus de 16 atomes de carbone ,ou végétales ou synthétiques, les silicones.
Selon un mode de réalisation, le ou les corps gras est ou sont choisis parmi l'huile de vaseline, les polydécènes, les esters d'acides gras ou d'alcools gras, liquides ou leurs mélanges, en particulier, le ou les corps gras de l'émulsion selon l'invention sont non siliconés.

Encore plus préférentiellement le ou les corps gras sont choisis parmi des huiles
L'émulsion (A) selon l'invention comprend une quantité supérieure à 25 % de corps gras. De préférence la concentration en corps gras va de 25 à 80 %, encore plus préférentiellement de 25 à 65 %, mieux de 30 à 55 % du poids total de l'émulsion. Selon un mode de réalisation particulier, l'émulsion contient parmi les corps gras, une ou plusieurs huiles. A titre d'exemple, on peut citer l'huile de vaseline, les polydécènes, les esters d'acides gras ou d'alcools gras liquides.

L'émulsion (A) comprend également un ou plusieurs tensioactifs non ioniques mono ou poly- oxyalkylénés à motifs oxyéthylénés choisis parmi les alcools oxyéthylénés en C18-C30 avec un nombre de motifs d'oxyde d'éthylène compris entre 1 et 50, de préférence entre 2 et 30. Les tensioactifs non ioniques ne comprennent pas de motifs oxypropylénés.

Comme alcools gras éthoxylés, on peut citer par exemple les produits d'addition d'oxyde d'éthylène avec l'alcool laurylique, notamment ceux comportant de 9 à 50 groupes oxyéthylénés et plus particulièrement ceux comportant de 10 à 12 groupes oxyéthylénés (Laureth-10 à Laureth-12 en noms CTFA) ; les produits d'addition d'oxyde d'éthylène avec l'alcool béhénylique, notamment ceux de 9 à 50 groupes oxyéthylénés (Beheneth-9 à Beheneth-50 en noms CTFA), de préférence 10 groupes oxyéthylénés. (Beheneth-10) ; les produits d'addition d'oxyde d'éthylène avec l'alcool cétéarylique (mélange d'alcool cétylique et d'alcool stéarylique), notamment ceux comportant de 10 à 30 groupes oxyéthylénés (Ceteareth-10 à Ceteareth-30 en noms CTFA) ;; les produits d'addition d'oxyde d'éthylène avec l'alcool stéarylique, notamment ceux comportant de 10 à 30 groupes oxyéthylénés (Steareth-10 à Steareth-30 en noms CTFA) ; les produits d'addition d'oxyde d'éthylène avec l'alcool isostéarylique, notamment ceux comportant de 10 à 50 groupes oxyéthylénés (Isosteareth-10 à Isosteareth-50 en noms CTFA) ; et leurs mélanges.

On peut utiliser aussi des mélanges de ces dérivés oxyéthylénés d'alcools gras et d'acides gras.

Comme acides gras éthoxylés, on peut citer par exemple les produits d'addition d'oxyde d'éthylène avec les acides laurique, palmitique, stéarique ou béhénique, et leurs mélanges, notamment ceux comportant de 9 à 50 groupes oxyéthylénés tels que les laurates de PEG-9 à PEG-50 (en noms CTFA : PEG-9 laurate à PEG-50 laurate) ; les palmitates de PEG-9 à PEG-50 (en noms CTFA : PEG-9 palmitate à PEG-50 palmitate) ; les stéarates de PEG-9 à PEG-50 (en noms CTFA : PEG-9 stearate à PEG-50 stearate) ; les palmito-stéarates de PEG-9 à PEG-50 ; les béhénates de PEG-9 à PEG-50 (en noms CTFA : PEG-9 behenate à PEG-50 behenate) ; et leurs mélanges.

Selon un mode préféré de réalisation, l'émulsion (A) comprend de préférence au moins l'alcool béhénylique.

De préférence, le tensioactif présent dans l'émulsion est un tensioactif non ionique oxyéthyléné présentant une HLB de 8 à 18. Le HLB est le rapport entre la partie hydrophile et la partie lipophile dans leur molécule. Ce terme HLB est bien connu de l'homme du métier et est décrit dans "The HLB system. A time-saving guide to Emulsifier Selection" (published by ICI Americas Inc; 1984).

Dans une variante préférée la composition ne contient pas de tensioactifs glycérolés.

La teneur en tensioactifs dans l'émulsion (A) représente plus particulièrement de 0,1 à 50 % en poids, de préférence de 0,5 à 30 % en poids par rapport au poids de l'émulsion.

L'émulsion (A) comprend un ou plusieurs agents oxydants. Plus particulièrement, le ou les agents oxydants sont choisis parmi le peroxyde d'hydrogène, le peroxyde d'urée, les bromates ou ferricyanures de métaux alcalins, les sels peroxygénés comme par exemple les persulfates, les perborates, les peracides et leurs précurseurs et les percarbonates de métaux alcalins ou alcalino-terreux.

Cet agent oxydant est avantageusement constitué par du peroxyde d'hydrogène et notamment en solution aqueuse (eau oxygénée) dont le titre peut varier, plus particulièrement, de 1 à 40 volumes, et encore plus préférentiellement de 5 à 40 volumes.

En fonction du degré d'éclaircissement souhaité, l'agent oxydant peut également comprendre un agent oxydant choisi de préférence parmi les sels peroxygénés .

L'émulsion (A) comprend de l'eau en quantité supérieure à 5 % en poids du poids total de l'émulsion, de préférence plus de 10 % en poids, et de manière encore plus avantageuse plus de 20 % en poids.

L'émulsion (A) peut également renfermer divers adjuvants utilisés classiquement dans les compositions pour l'éclaircissement des cheveux, tels que des polymères anioniques, cationiques, non ioniques, amphotères, zwitterioniques ou leurs mélanges ; des agents épaississants minéraux, et en particulier des charges telles que des argiles, le talc ; des agents épaississants organiques, avec en particulier les épaississants associatifs polymères anioniques, cationiques, non ioniques et amphotères ; des agents antioxydants ; des agents de pénétration ; des agents séquestrants ; des parfums ; des agents dispersants ; des agents filmogènes ; des agents conservateurs ; des agents opacifiants.

Elle peut éventuellement comprendre un solvant organique. A titre de solvant organique, on peut par exemple citer, les alcanols, linéaires ou ramifiés, en C₂-C₄, tels que l'éthanol et l'isopropanol ; le glycérol ; les polyols et éthers de polyols comme le 2-butoxyéthanol, le propylèneglycol, le dipropylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Selon un mode de réalisation particulier, l'émulsion (A) peut être préparée par des procédés de préparation classique d'émulsion directe mais aussi par un procédé par PIT. De préférence l'émulsion directe oxydante est préparée par un procédé PIT.

Selon ce mode de réalisation particulier, le principe d'émulsification par inversion de phase en température (en Anglais : Phase Inversion Temperature ou PIT) est, dans son principe, bien connu de l'homme de l'art ; il a été décrit en 1968 par K. Shinoda (J. Chem. Soc. Jpn., 1968, 89, 435). Il a été montré que cette technique d'émulsification permet d'obtenir des émulsions fines stables (K. Shinoda et H. Saito, J. Colloïd Interface Sci., 1969,30, 258). Cette technologie a été appliquée en cosmétique dès 1972 par Mitsui et al. («Application of the phase-inversion-temperature method to the emulsification of cosmetics» ; T. Mitsui, Y. Machida and F. Harusawa, American. Cosmet. Perfum., 1972, 87, 33).

Le principe de cette technique est le suivant : on réalise le mélange d'une phase aqueuse et d'une phase huileuse, que l'on porte à une température supérieure à la température PIT, température d'inversion de phase du système, qui est la température à laquelle l'équilibre entre les propriétés hydrophile et lipophile du ou des émulsionnants mis en oeuvre est atteint ; à température élevée, c'est-à-dire supérieure à la température d'inversion de phase (>PIT), l'émulsion est de type eau-dans-huile, et, au cours de son refroidissement, cette émulsion s'inverse à la température d'inversion de phase, pour devenir une émulsion de type huile-dans-eau, et ceci en étant passée auparavant par un état de microémulsion. Ce procédé permet d'obtenir facilement des émulsions de diamètre inférieur à 4 µm.

Selon ce procédé PIT, l'émulsion directe (A) objet de l'invention comprend une émulsion directe (huile dans eau) qui comprend au moins 25 % d'un ou plusieurs corps gras dont au moins une huile, un ou plusieurs tensio-actifs dont au moins un est un tensio-actif non ionique présentant un point de trouble, une quantité d'eau supérieure à 5 % en poids, du poids total de l'émulsion. Selon ce mode de réalisation particulier, le tensio-actif non ionique présente un HLB compris entre 8 et 18. Il est de préférence choisi parmi les tensioactifs oxyalkylénés, de préférence oxyéthylénés tels que les alcools gras éthoxylés, les acides gras éthoxylés, les glycérides partiels d'acides gras éthoxylés, les triglycérides d'acides gras polyglycérolés et leurs dérivés éthoxylés, et leurs mélanges. Par ailleurs, une telle émulsion présente une taille de particule inférieure à 4 µm, de préférence inférieure à 1µm.

De manière plus détaillée, on peut opérer de manière suivante pour obtenir une émulsion PIT :
1) Peser dans un récipient tous les constituants de l'émulsion directe (A)
2) Homogénéiser le mélange, par exemple au moyen d'un Rayneri 350 tours/min, et chauffer en augmentant progressivement la température au moyen d'un bain marie jusqu'à une température supérieure à la température d'inversion de phase T1, c'est-à-dire jusqu'à l'obtention d'une phase transparente ou translucide (zone de microémulsion ou de phase lamellaire) puis d'une phase plus visqueuse qui indique l'obtention de l'émulsion inverse (E/H).
3) Arrêter le chauffage et maintenir l'agitation jusqu'à retour à la température ambiante, en passant par la température d'inversion de phase T1, c'est-à-dire la température à laquelle se forme une émulsion H/E fine.
4) Lorsque la température est redescendue en dessous de la zone d'inversion de Phase en Température (T1), additionner les éventuels additifs et les matières premières thermosensibles.

On obtient une composition finale stable dont les gouttelettes de phase lipophile sont fines avec des tailles de 10 à 200 nm.

Dans la zone de formation d'une microémulsion (mélange translucide), les interactions hydrophiles et hydrophobes sont équilibrées car la tendance du tensioactif est de former aussi bien des micelles directes que des micelles inverses. Par chauffage au-delà de cette zone, il y a formation d'une émulsion E/H, car le tensioactif favorise la formation d'une émulsion eau dans huile. Puis lors du refroidissement en dessous de la zone d'inversion de phase, l'émulsion devient une émulsion directe (H/E).

L'émulsification par inversion de phase est expliquée en détail dans l'ouvrage T.Fôrster, W von Rybinski, A.Wadle, Influence of microemulsion phases on the préparation of fine disperse emulsions, Advances in Colloid and interface sciences, 58, 119-149, 1995 cité ici pour référence.

La composition B comprend un ou plusieurs agents alcalins choisis parmi les amines organiques.

A titre d'exemple d'amine organique, on peut citer les amines organiques comprenant une ou deux fonctions amine primaire, secondaire ou tertiaire, et un ou plusieurs groupements alkyle, linéaires ou ramifiés, en C₁-C₈ porteurs d'un ou plusieurs radicaux hydroxyle.

Conviennent en particulier à la réalisation de l'invention les amines organiques choisies parmi les alcanolamines telles que les mono-, di- ou tri- alcanolamines, comprenant un à trois radicaux hydroxyalkyle, identiques ou non, en C₁-C₄.

Parmi des composés de ce type, on peut citer la monoéthanolamine, la diéthanolamine, la triéthanolamine, la monoisopropanolamine, la diisopropanolamine, la N-diméthylaminoéthanolamine, le 2-amino-2-méthyl-1-propanol, la triisopropanolamine, le 2-amino-2-méthyl-1,3-propanediol, le 3-amino-1,2-propanediol, le 3-diméthylamino-1,2-propanediol, le tris-hydroxyméthylaminométhane.

Conviennent également les amines organiques de formule suivante : 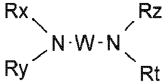 dans laquelle W est un reste alkylène en C₁-C₆ éventuellement substitué par un groupement hydroxyle ou un radical alkyle en C₁-C₆ ; Rx, Ry, Rz et Rt, identiques ou différents, représentent un atome d'hydrogène, un radical alkyle en C₁-C₆ ou hydroxyalkyle en C₁-C₆, aminoalkyle en C₁-C₆.

On peut citer à titre d'exemple de telles amines, le 1,3 diaminopropane, le 1,3 diamino 2 propanol, la spermine, la spermidine.

Selon une autre variante de l'invention, l'amine organique est choisie parmi les acides aminés.

Plus particulièrement, les acides aminés utilisables sont d'origine naturelle ou de synthèse, sous leur forme L, D, ou racémique et comportent au moins une fonction acide choisie plus particulièrement parmi les fonctions acides carboxyliques, sulfoniques, phosphoniques ou phosphoriques. Les acides aminés peuvent se trouver sous forme neutre ou ionique.

De manière avantageuse, les acides aminés sont des acides aminés basiques comprenant une fonction amine supplémentaire éventuellement incluse dans un cycle ou dans une fonction uréido.

De tels acides aminés basiques sont choisis de préférence parmi ceux répondant à la formule (I) suivante : 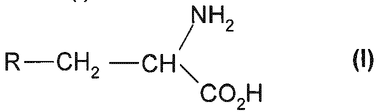 où R désigne un groupe choisi parmi :

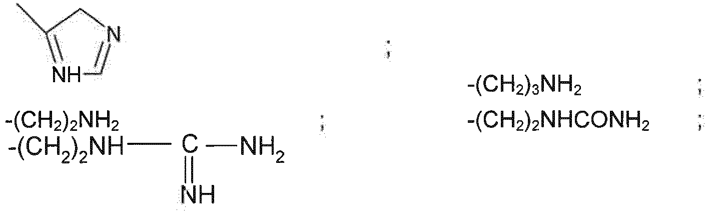

Les composés correspondants à la formule (I) sont l'histidine, la lysine, l'arginine, l'ornithine, la citrulline.

A titre d'acides aminés utilisables dans la présente invention, on peut notamment citer l'acide aspartique, l'acide glutamique, l'alanine, l'arginine, l'ornithine, la citrulline, l'asparagine, la carnitine, la cystéine, la glutamine, la glycine, l'histidine, la lysine, l'isoleucine, la leucine, la méthionine, la N-phénylalanine, la proline, la serine, la taurine la thréonine, le tryptophane, la tyrosine et la valine.

Selon une variante préférée de l'invention, l'amine organique est choisie parmi les acides aminés basiques. Les acides aminés particulièrement préférés sont l'arginine, la lysine, l'histidine, ou leurs mélanges.

Selon une autre variante de l'invention, l'amine organique est choisie parmi les amines organiques de type hétérocycliques On peut en particulier citer, outre l'histidine déjà mentionnée dans les acides aminés, la pyridine, la pipéridine, l'imidazole, le 1,2,4-triazole, le tétrazole, le benzimidazole .

Selon une autre variante de l'invention, l'amine organique est choisie parmi les dipeptides d'acides aminés. A titre de dipeptides d'acides aminés utilisables dans la présente invention, on peut notamment citer la carnosine, l'anserine et la baleine

Selon une autre variante de l'invention, l'amine organique est choisie parmi les composés comportant une fonction guanidine. A titre d'amines d'amines de ce type utilisables dans la présente invention, on peut notamment citer outre l'arginine déjà mentionnée à titre d'acide aminé, la créatine, la créatinine, la 1,1-diméthylguanidine, 1,1-diéthylguanidine, la glycocyamine, la metformin, l'agmatine, la n-amidinoalanine, l'acide 3-guanidinopropionique, l'acide 4-guanidinobutyrique et l'acide 2-([amino(imino)methyl]amino)ethane-1-sulfonique.

De préférence l'amine organique est une alcanolamine. Plus préférentiellement l'amine organique est choisie parmi le 2-amino 2-méthyl 1-propanol, la monoéthanolamine ou leurs mélanges. Encore plus préférentiellement l'amine organique est la monoéthanolamine.

L'agent alcalin peut être une amine organique sous forme de sels. Par sel d'amine organique, on entend au sens de la présente invention, les sels organiques ou inorganiques d'une amine organique telle que décrite ci-dessus.

De préférence, les sels organiques sont choisis parmi les sels d'acides organiques tels que les citrates, les lactates, les glycolates, les gluconates, les acétates, les propionates, les fumarates, les oxalates et les tartrates.

De préférence, les sels inorganiques sont choisis parmi les halogénohydrates (chlorhydrates par exemple), les carbonates, les hydrogénocarbonates, les sulfates, les hydrogénophosphates et les phosphates.

Selon un mode de réalisation particulier, la composition contient en tant qu'agents alcalins au moins une alkanolamine. Lorsque la composition contient plusieurs agents alcalins dont une alkanolamine et de l'ammoniaque ou un de ses sels, la ou les amines organiques sont de préférence majoritaire en poids par rapport à la quantité ammoniac.

Généralement, la composition (B) présente une teneur en agents alcalins allant de 0,1 à 40 % en poids, de préférence de 0,5 à 20 % en poids par rapport au poids de ladite composition.

Cette composition (B) peut également comprendre un ou plusieurs solvants organiques tels que décrits précédemment. Elle peut aussi comprendre un ou plusieurs agents acidifiants.

La composition B peut comprendre un ou plusieurs colorants. Ce ou Ces colorants peuvent être des colorants directs ou d'oxydation.

Les colorants d'oxydation sont en général choisis parmi les bases d'oxydation éventuellement combinée(s) à un ou plusieurs coupleurs.

A titre d'exemple, les bases d'oxydation sont choisies parmi les paraphénylènediamines, les bis-phénylalkylènediamines, les para-aminophénols, les ortho-aminophénols, les bases hétérocycliques et leurs sels d'addition.

Parmi les paraphénylènediamines, on peut citer à titre d'exemple, la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylène-diamine, la 4-aminophénylpyrrolidine, la 2-thiényl paraphénylènediamine, le 2-β hydroxyéthylamino 5-amino toluène, la 3-hydroxy 1-(4'-aminophényl)pyrrolidine et leurs sels d'addition avec un acide.

Parmi les paraphénylènediamines citées ci-dessus, la paraphénylènediamine, la paratoluylènediamine, la 2-isopropyl paraphénylènediamine, la 2-β-hydroxyéthyl paraphénylènediamine, la 2-β-hydroxyéthyloxy paraphénylène-diamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, et leurs sels d'addition avec un acide sont particulièrement préférées.

Parmi les bis-phénylalkylènediamines, on peut citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diamino phénoxy)-3,6-dioxaoctane, et leurs sels d'addition.

Parmi les para-aminophénols, on peut citer à titre d'exemple, le para-aminophénol, le 4-amino 3-méthyl phénol, le 4-amino 3-fluoro phénol, le 4-amino-3-chlorophénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les ortho-aminophénols, on peut citer à titre d'exemple, le 2-amino phénol, le 2-amino 5-méthyl phénol, le 2-amino 6-méthyl phénol, le 5-acétamido 2-amino phénol, et leurs sels d'addition.

Parmi les bases hétérocycliques, on peut citer à titre d'exemple, les dérivés pyridiniques, les dérivés pyrimidiniques et les dérivés pyrazoliques.

Parmi les dérivés pyridiniques, on peut citer les composés décrits par exemple dans les brevets GB 1 026 978 et GB 1 153 196, comme la 2,5-diamino pyridine, la 2-(4-méthoxyphényl)amino 3-amino pyridine, la 3,4-diamino pyridine, et leurs sels d'addition.

D'autres bases d'oxydation pyridiniques utiles dans la présente invention sont les bases d'oxydation 3-amino pyrazolo-[1,5-a]-pyridines ou leurs sels d'addition décrits par exemple dans la demande de brevet FR 2801308. A titre d'exemple, on peut citer la pyrazolo[1,5-a]pyridin-3-ylamine ; la 2-acétylamino pyrazolo-[1,5-a] pyridin-3-ylamine ; la 2-morpholin-4-yl-pyrazolo[1,5-a]pyridin-3-ylamine ; l'acide 3-amino-pyrazolo[1,5-a]pyridin-2-carboxylique ; la 2-méthoxy-pyrazolo[1,5-a]pyridine-3-ylamino ; le (3-amino-pyrazolo[1,5-a]pyridine-7-yl)-méthanol ; le 2-(3-amino-pyrazolo[1,5-alpyridine-5-yl)-éthanol ; le 2-(3-amino-pyrazolo[1,5-a]pyridine-7-yl)-éthanol ; le (3-amino-pyrazolo[1,5-a]pyridine-2-yl)-méthanol ; la 3,6-diamino-pyrazolo[1,5-a]pyridine ; la 3,4-diamino-pyrazolo[1,5-a]pyridine ; la pyrazolo[1,5-a]pyridine-3,7-diamine ; la 7-morpholin-4-yl-pyrazolo[1,5-a]pyridin-3-ylamine ; la pyrazolo[1,5-a]pyridine-3,5-diamine ; la 5-morpholin-4-yl-pyrazolo[1,5-a]pyridin-3-ylamine ; le 2-[(3-amino-pyrazolo[1,5-a]pyridin-5-yl)-(2-hydroxyéthyl)-amino]-éthanol ; le 2-[(3-amino-pyrazolo[1,5-a]pyridin-7-yl)-(2-hydroxyéthyl)-amino]-éthanol ; la 3-amino-pyrazolo[1,5-a]pyridine-5-ol ; 3-amino-pyrazolo[1,5-a]pyridine-4-ol ; la 3-amino-pyrazolo[1,5-a]pyridine-6-ol ; la 3-amino-pyrazolo[1,5-a]pyridine-7-ol ; ainsi que leurs sels d'addition.

Parmi les dérivés pyrimidiniques, on peut citer les composés décrits par exemple dans les brevets DE 2359399 ; JP 88-169571 ; JP 05-63124 ; EP 0770375 ou demande de brevet WO 96/15765 comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine et leurs sels d'addition et leurs formes tautomères, lorsqu'il existe un équilibre tautomérique.

Parmi les dérivés pyrazoliques, on peut citer les composés décrits dans les brevets DE 3843892, DE 4133957 et demandes de brevet WO 94/08969, WO 94/08970, FR-A-2 733 749 et DE 195 43 988 comme le 4,5-diamino 1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition. On peut aussi utiliser le 4-5-diamino 1-(β-méthoxyéthyl)pyrazole.

A titre de base hétérocyclique,on peut aussi citer la 2,3 diamino 6,7 dihydro 1 H5H [pyrazolo1,2,a] pyrazol-1-one ou l'un de ses sels.

La composition cosmétique (B) selon l'invention peut éventuellement comprendre un ou plusieurs coupleurs choisis avantageusement parmi ceux conventionnellement utilisés pour la teinture des fibres kératiniques.

Parmi ces coupleurs, on peut notamment citer les méta-phénylènediamines, les méta-aminophénols, les méta-diphénols, les coupleurs naphtaléniques, les coupleurs hétérocycliques ainsi que leurs sels d'addition.

A titre d'exemple, on peut citer le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6- hydroxy benzomorpholine la 3,5-diamino-2,6-diméthoxypyridine, le 1-N-(β-hydroxyéthyl)amino-3,4-méthylène dioxybenzène, le 2,6-bis-(β-hydroxyéthylamino)toluène, la 6-hydroxy indoline, la 2,6-dihydroxy 4-méthyl pyridine, la 1-H 3-méthyl pyrazole 5-one, la 1-phényl 3-méthyl pyrazole 5-one, le 2,6-diméthyl pyrazolo [1,5-b]-1,2,4-triazole, le 2,6-diméthyl [3,2-c]-1,2,4-triazole, le 6-méthyl pyrazolo [1,5-a]-benzimidazole, leurs sels d'addition avec un acide, et leurs mélanges.

D'une manière générale, les sels d'addition des bases d'oxydation et des coupleurs utilisables dans le cadre de l'invention sont notamment choisis parmi les sels d'addition avec un acide tels que les chlorhydrates, les bromhydrates, les sulfates, les citrates, les succinates, les tartrates, les lactates, les tosylates, les benzènesulfonates, les phosphates et les acétates.

La ou les bases d'oxydation représentent chacune avantageusement de 0,0001 à 10 % en poids par rapport au poids total de la composition, et de préférence de 0,005 à 5 % en poids par rapport au poids total de la composition.

La teneur en coupleur(s), s'il(s) est(sont) présent(s), représentent chacun avantageusement de 0,0001 à 10 % en poids par rapport au poids total de la composition, et de préférence de 0,005 à 5 % en poids par rapport au poids total de la composition cosmétique (B).

En ce qui concerne les colorants directs, ces derniers sont plus particulièrement choisis parmi les espèces ioniques ou non ioniques, de préférence cationiques ou non ioniques.

A titre d'exemples de colorants directs convenables, on peut citer les colorants directs azoïques ; méthiniques ; carbonyles ; aziniques ; nitrés (hétéro)aryle ; tri-(hétéro)aryle méthanes ; les porphyrines ; les phtalocyanines et les colorants directs naturels, seuls ou en mélanges.

Plus particulièrement, les colorants azoïques comprennent une fonction - N=N- dont les deux atomes d'azote ne sont pas simultanément engagés dans un cycle. Il n'est toutefois pas exclu que l'un des deux atomes d'azote de l'enchaînement -N=N- soit engagé dans un cycle.

Les colorants de la famille des méthines sont plus particulièrement des composés comprenant au moins un enchaînement choisi parmi >C=C< et -N=C< dont les deux atomes ne sont pas simultanément engagés dans un cycle. Il est toutefois précisé que l'un des atomes d'azote ou de carbone des enchaînements peut être engagé dans un cycle. Plus particulièrement, les colorants de cette famille sont issus de composés de type méthine, azométhine, mono- et diarylméthane, indoamines (ou diphénylamines), indophénols, indoanilines, carbocyanines, azacabocyanines et leurs isomères, diazacarbocyanines et leurs isomères, tétraazacarbocyanines, hémicyanines

Concernant les colorants de la famille des carbonyles, on peut citer par exemple les colorants choisis parmi les acridone, benzoquinone, anthraquinone, naphtoquinone, benzanthrone, anthranthrone, pyranthrone, pyrazolanthrone, pyrimidinoanthrone, flavanthrone, idanthrone, flavone, (iso)violanthrone, isoindolinone, benzimidazolone, isoquinolinone, anthrapyridone, pyrazoloquinazolone, périnone, quinacridone, quinophthalone, indigoïde, thioindigo, naphtalimide, anthrapyrimidine, dicétopyrrolopyrrole, coumarine.

Concernant les colorants de la famille des azines cycliques, on peut citer notamment les azine, xanthène, thioxanthène, fluorindine, acridine, (di)oxazine, (di)thiazine, pyronine.

Les colorants nitrés (hétéro)aromatiques sont plus particulièrement des colorants directs nitrés benzéniques ou nitrés pyridiniques.

Concernant les colorants de type porphyrines ou phtalocyanines, on peut mettre en oeuvre des composés cationiques ou non, comprenant éventuellement un ou plusieurs métaux ou ions métalliques, comme par exemple des métaux alcalins et alcalino-terreux, le zinc et le silicium.

A titre d'exemple de colorants directs particulièrement convenables, on peut citer les colorants nitrés de la série benzénique ; les colorants directs azoïques ; azométhiniques ; méthiniques ; les azacarbocyanines comme les tétraazacarbocyanines (tétraazapentaméthines) ; les colorants directs quinoniques et en particulier anthraquinoniques, naphtoquinoniques ou benzoquinoniques ; les colorants directs aziniques ; xanthéniques ; triarylméthaniques ; indoaminiques ; indigoïdes ; phtalocyanines, porphyrines et les colorants directs naturels, seuls ou en mélanges.

Ces colorants peuvent être des colorants monochromophoriques (c'est-à-dire ne comprenant qu'un seul colorant) ou polychromophoriques, de préférence di- ou tri- chromophoriques ; les chromophores pouvant être identiques ou non, de la même famille chimique ou non. A noter que qu'un colorant polychromophorique comprend plusieurs radicaux issus chacun d'une molécule absorbant dans le domaine visible entre 400 et 800 nm. De plus cette absorbance du colorant ne nécessite ni oxydation préalable de celui-ci, ni association avec d'autre(s) espèce(s) chimique(s).

Dans le cas de colorants polychromophoriques, les chromophores sont reliés entre eux au moyen d'au moins un bras de liaison qui peut être cationique ou non.

Parmi les colorants directs benzéniques utilisables selon l'invention, on peut citer de manière non limitative les composés suivants :
- 1,4-diamino-2-nitrobenzène,
- 1-amino-2nitro-4-β-hydroxyéthylaminobenzène
- 1-amino-2nitro-4-bis(β-hydroxyéthyl)-aminobenzène
- 1,4-bis(β-hydroxyéthylamino)-2-nitrobenzène
- 1-β-hydroxyéthylamino-2-nitro-4-bis-(β-hydroxyéthylamino)-benzène
- 1-β-hydroxyéthylamino-2-nitro-4-aminobenzène
- 1-β-hydroxyéthylamino-2-nitro-4-(éthyl)(β-hydroxyéthyl)-aminobenzène
- 1-amino-3-méthyl-4-β-hydroxyéthylamino-6-nitrobenzène
- 1-amino-2-nitro-4-β-hydroxyéthylamino-5-chlorobenzène
- 1,2-diamino-4-nitrobenzène
- 1-amino-2-β-hydroxyéthylamino-5-nitrobenzène
- 1,2-bis-(β-hydroxyéthylamino)-4-nitrobenzène
- 1-amino-2-tris-(hydroxyméthyl)-méthylamino-5-nitrobenzène
- 1-Hydroxy-2-amino-5-nitrobenzène
- 1-Hydroxy-2-amino-4-nitrobenzène
- 1-Hydroxy-3-nitro-4-aminobenzène
- 1-Hydroxy-2-amino-4,6-dinitrobenzène
- 1-β-hydroxyéthyloxy-2-β-hydroxyéthylamino-5-nitrobenzène
- 1-Méthoxy-2-β-hydroxyéthylamino-5-nitrobenzène
- 1-β-hydroxyéthyloxy-3-méthylamino-4-nitrobenzène
- 1-β,γ-dihydroxypropyloxy-3-méthylamino-4-nitrobenzène
- 1-β-hydroxyéthylamino-4-β,γ-dihydroxypropyloxy-2-nitrobenzène
- 1-β,γ-dihydroxypropylamino-4-trifluorométhyl-2-nitrobenzène
- 1-β-hydroxyéthylamino-4-trifluorométhyl-2-nitrobenzène
- 1-β-hydroxyéthylamino-3-méthyl-2-nitrobenzène
- 1-β-aminoéthylamino-5-méthoxy-2-nitrobenzène
- 1-Hydroxy-2-chloro-6-éthylamino-4-nitrobenzène
- 1-Hydroxy-2-chloro-6-amino-4-nitrobenzène
- 1-Hydroxy-6-bis-(β-hydroxyéthyl)-amino-3-nitrobenzène
- 1-β-hydroxyéthylamino-2-nitrobenzène
- 1-Hydroxy-4-β-hydroxyéthylamino-3-nitrobenzène.

Parmi les colorants directs azoïques, azométhines, méthines ou tétraazapentaméthines utilisables selon l'invention on peut citer les colorants cationiques décrits dans les demandes de brevets WO 95/15144, WO 95/01772 et EP 714954 ; FR 2189006, FR 2285851, FR-2140205, EP 1378544, EP 1674073.

Parmi on pet aussi citer les composés suivants :

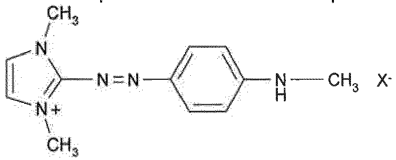

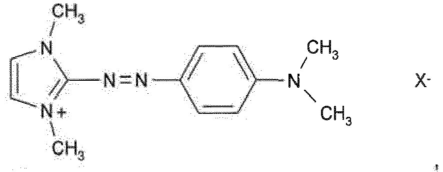

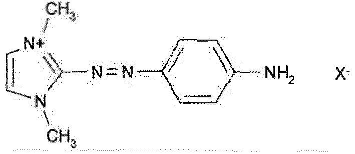

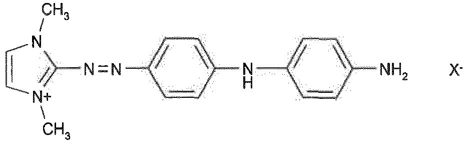

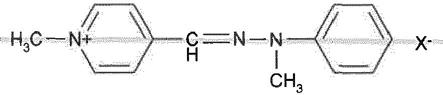

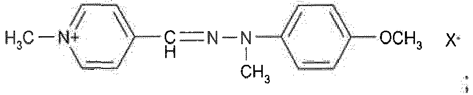

On peut également citer parmi les colorants directs azoïques les colorants suivants, décrits dans le COLOUR INDEX INTERNATIONAL 3e édition :
- Disperse Red 17
- Basic Red 22
- Basic Red 76
- Basic Yellow 57
- Basic Brown 16
- Basic Brown 17
- Disperse Black 9.

On peut aussi citer le 1-(4'-aminodiphénylazo)-2-méthyl-4bis-(β-hydroxyéthyl) aminobenzène.

Parmi les colorants directs quinoniques on peut citer les colorants suivants :
- Disperse Red 15
- Solvent Violet 13
- Disperse Violet 1
- Disperse Violet 4
- Disperse Blue 1
- Disperse Violet 8
- Disperse Blue 3
- Disperse Red 11
- Disperse Blue 7
- Basic Blue 22
- Disperse Violet 15
- Basic Blue 99
ainsi que les composés suivants :
- 1-N-méthylmorpholiniumpropylamino-4-hydroxy anthraquinone
- 1-Aminopropylamino-4-méthylaminoanthraquinone
- 1-Aminopropylaminoanthraquinone
- 5-β-hydroxyéthyl-1,4-diaminoanthraquinone
- 2-Aminoéthylaminoanthraquinone
- 1,4-Bis-(β,γ-dihydroxypropylamino)-anthraquinone.

Parmi les colorants aziniques, on peut citer les composés suivants :
- Basic Blue 17
- Basic Red 2.

Parmi les colorants triarylméthaniques utilisables selon l'invention, on peut citer les composés suivants :
- Basic Green 1
- Basic Violet 3
- Basic Violet 14
- Basic Blue 7
- Basic Blue 26

Parmi les colorants indoaminiques utilisables selon l'invention, on peut citer les composés suivants :
- 2-β-hydroxyéthlyamino-5-[bis-(β-4'-hydroxyéthyl)amino]anilino-1,4-benzoquinone
- 2-β-hydroxyéthylamino-5-(2'-méthoxy-4'-amino)anilino-1,4-benzoquinone
- 3-N(2'-Chloro-4'-hydroxy)phényl-acétylamino-6-méthoxy-1,4-benzoquinone imine
- 3-N(3'-Chloro-4'-méthylamino)phényl-uréido-6-méthyl-1,4-benzoquinone imine
- 3-[4'-N-(Ethyl,carbamylméthyl)-amino]-phényl-uréido-6-méthyl-1,4-benzoquinone imine.

Parmi les colorants de type tétraazapentaméthiniques utilisables selon l'invention, on peut citer les composés suivants figurant dans le tableau ci-dessous. An étant défini comme précédemment :

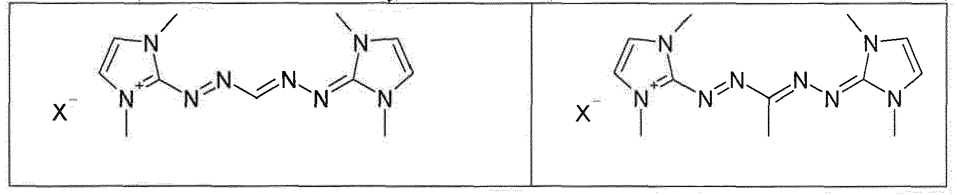

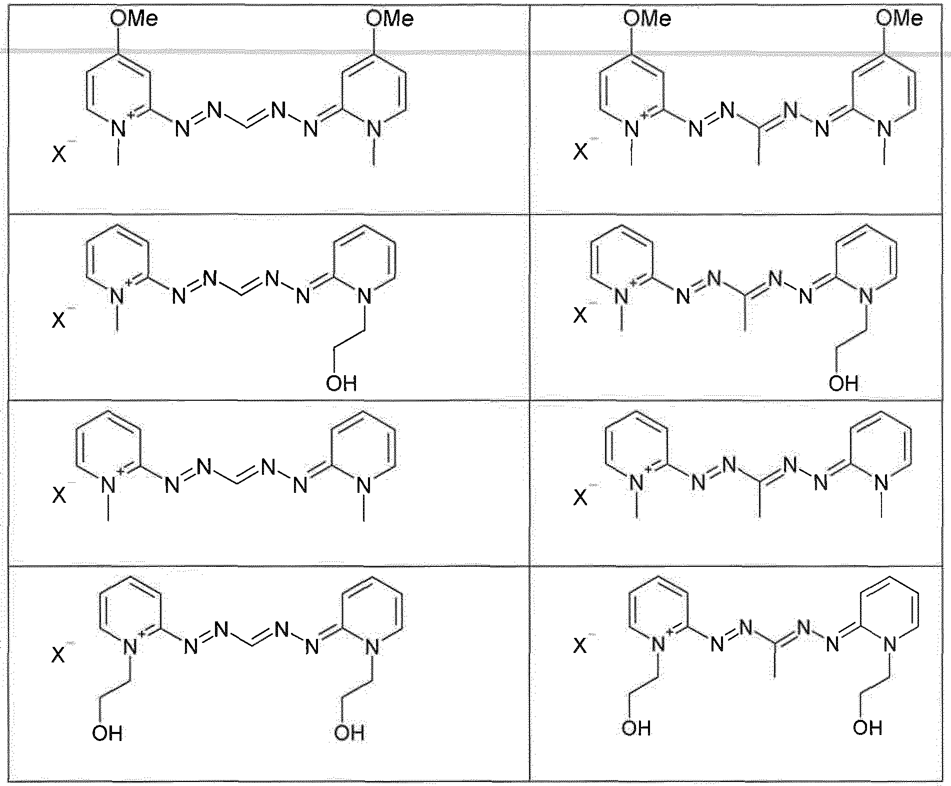

X⁻ représente un anion de préférence choisi parmi le chlorure, l'iodure, le méthyl sulfate, l'éthyl sulfate, l'acétate et le perchlorate.

Parmi les colorants polychromophoriques, on peut citer plus particulièrement se reporter aux demandes de brevets EP 1637566, EP 1619221, EP 1634926, EP 1619220, EP 1672033, EP 1671954, EP 1671955, EP 1679312, EP 1671951, EP167952, EP167971, WO 06/063866, WO 06/063867, WO 06/063868, WO 06/063869, EP 1408919, EP 1377264, EP 1377262, EP 1377261, EP 1377263, EP 1399425, EP 1399117, EP 1416909, EP 1399116, EP 1671560.

On peut aussi également mettre en oeuvre des colorants directs cationiques cités dans les demandes EP 1006153, qui décrit des colorants comprenant deux chromophores de type anthraquinones reliés au moyen d'un bras de liaison cationique ; EP 1433472, EP 1433474, EP 1433471 et EP 1433473 qui décrivent des colorants dichromophoriques identiques ou non, reliés par un bras de liaison cationique ou non, ainsi que EP 6291333 qui décrit notamment des colorants comprenant trois chromophores, l'un d'eux étant un chromophore anthraquinone auquel sont reliés deux chromophores de type azoïque ou diazacarbocyanine ou l'un de ses isomères.

Parmi les colorants directs naturels utilisables selon l'invention, on peut citer la lawsone, la juglone, l'alizarine, la purpurine, l'acide carminique, l'acide kermésique, la purpurogalline, le protocatéchaldéhyde, l'indigo, l'isatine, la curcumine, la spinulosine, l'apigénidine, les orcéines. On peut également utiliser les extraits ou décoctions contenant ces colorants naturels et notamment les cataplasmes ou extraits à base de henné.

Lorsqu'ils sont présents, le ou les colorants directs représentent plus particulièrement de 0,0001 à 10 % en poids du poids total de la composition, et de préférence de 0,005 à 5 % en poids.

Enfin, la composition (B) se présente sous diverses formes, comme par exemple une poudre, une solution, une émulsion ou un gel.

La composition B peut être anhydre.

Si la composition B contient ou plusieurs colorants elle est de préférence aqueuse.
Si la composition B est anhydre, elle peut comprendre des composés peroxygénés (JC) tel que le peroxyde d'urée , les sels peroxygénés comme par exemple les persulfates, les perborates, les peracides et leurs précurseurs, et les percarbonates de métaux alcalins ou alcalino-terreux. Dans ce cas elle peut notamment être utilisée dans les procédés d'éclaircissement des fibres kératiniques.

Le procédé de l'invention peut être mis en oeuvre en appliquant sur les matières kératiniques l'émulsion (B) et la composition (A) successivement et sans rinçage intermédiaire, l'ordre étant indifférent..

La composition (B) peut contenir d'autres additifs, par exemple les additifs décrits pour l'émulsion (A). Selon une variante, l"émulsion (A) et/ou la composition (B) comprennent un épaississant. A titre d'épaississant, on peut citer les carbomers, les polymères carboxyvinyliques à groupements hydrophobes, les épaississants à motifs sucres, les polymères amphiphiles statistiques d'AMPS modifiés par réaction avec une n-monoalkylamine ou une di-n-alkylamine en C6-C22, et tels que ceux décrits dans la demande de brevet WO00/31154. Ces polymères peuvent également contenir d'autres monomères hydrophiles éthyléniquement insaturés choisis par exemple parmi les acides (méth)acryliques, leurs dérivés alkyl substitués en β ou leurs esters obtenus avec des monoalcools ou des mono- ou poly- alkylèneglycols, les (méth)acrylamides, la vinylpyrrolidone, l'anhydride maléique, l'acide itaconique ou l'acide maléique ou les mélanges de ces composés.

Selon un mode de réalisation du procédé de l'invention, on applique sur les matières kératiniques, sèches ou humides, une composition obtenue par mélange extemporané, au moment de l'emploi, de l'émulsion (A) et de la composition (B). Selon ce mode de réalisation, le rapport pondéral des quantités de (A) / (B) et R2 varie de 0,1 à 10 de préférence de 0,2 à 2, mieux de 0,3 à 1.

En outre, indépendamment de la variante mise en oeuvre, le mélange présent sur les matières kératiniques (résultant soit du mélange extemporané de (A) et (B) ou de leur application successive partielle ou totale) est laissé en place pour une durée, en général, de l'ordre de 1 minute à 1 heure, de préférence de 5 minutes à 30 minutes.

La température durant le procédé est classiquement comprise entre la température ambiante (entre 15 à 25°C) et 80°C, de préférence entre la température ambiante et 60°C.

A l'issue du traitement, les matières kératiniques sont éventuellement rincées à l'eau, subissent éventuellement un lavage suivi d'un rinçage à l'eau, avant d'être séchées ou laissées à sécher.

Selon une variante, la composition de coloration obtenue après mélange de l'émulsion (A) décrite précédemment et de la composition aqueuse (B) comprenant un agent oxydant est telle que après mélange la quantité de corps gras est supérieure à 20 %, de préférence supérieure à 25 %, voire supérieure à 30 %.

Enfin, l'invention concerne un dispositif à plusieurs compartiments comprenant dans un premier compartiment une émulsion (A), et dans un second, une composition (B) comprenant un ou plusieurs agents alcalins, ces compositions ayant été décrites auparavant.

De préférence les matières kératiniques sont des cheveux humains.

### EXEMPLES

On prépare les compositions suivantes :

### Exemples de l'invention

La composition A est préparée au moyen d'un procédé par PIT.

**Composition A1**

| | Nom INCI | g% |
|---|---|---|
| **Emulsion A** | BEHENETH-10 | 6,00 |
| | Sorbitol | 5,00 |
| | Huile de vaseline | 61,5722 |
| | Eau | 10,00 |
| | Ethanol | 2,00 |
| | Pyrophosphate de sodium | 0,04 |
| | Salicylate de sodium | 0,035 |
| | Acide étidronique | 0,2 |
| | Poloxamer 184 | 3,00 |
| | Acide phosphorique qs pH 3 | |
| | Peroxyde d'hydrogène | 6 |

### Procédé de fabrication de l'émulsion A1:

1- On chauffe les ingrédients de l'émulsion A au bain marie sous agitation du type Rayneri (400 tr/min). On obtient une émulsion blanche fluide qui devient translucide vers 68°C et s'épaissit au-delà.
2- Dès que l'émulsion s'épaissit, on retire le bain marie et on laisse refroidir sous même agitation.
3- Vers 50°C, on introduit le poloxamer 184.
4- A Température ambiante, on introduit l'acide, les séquestrants puis l'eau oxygénée et on réajuste l'eau perdue à l'évaporation (<5%).

On obtient ainsi une émulsion gélifiée translucide de taille de gouttes < 1 µm (viscosité 63 UD M3 Rheomat, pH = 3)

La composition B comprenant l'agent alcalin est obtenue par le mélange d'une base anhydre B1 et d'une composition à base de Monoéthanolamine B2 dans les proportions de 10g de B1 pour 4g de B2.

**Base anhydre B1**

| Nom | Concentration en % |
|---|---|
| **Huile de vaseline** | **64,5** |
| **Octyl dodécanol** | **11,5** |
| **Bentone** | **3** |
| **Propylène Glycol** | **1** |
| **Monolaurate de Sorbitane Oxyéthyléné (2OE)** | **11** |
| **Acide laurylique oxyéthyléné (2OE)** | **1** |
| **stéarate de glycol** | **8** |

**Composition B2**

| Nom | Concentration en % |
|---|---|
| **propylène glycol** | **6,2** |
| **alcool éthylique** | **8,8** |
| **héxylène glycol** | **3** |
| **dipropylène glycol** | **3** |
| **monoéthanolamine** | **14,5** |
| **Poloxamer 124** | **60,25** |
| **Acide ascorbique** | **0,25** |
| **silice pyrogénée à carctère hydrophobe** | **4** |

L'émulsion A1 est mélangée à la composition B, dans un rapport 15/14. Le mélange est ensuite appliqué sur des cheveux naturels châtains (hauteur de ton = 4). Le rapport de bain « mélange/mèche » est respectivement de 10/1 (g/g). Le temps de pose est de 30min à 27°C. A l'issue de ce temps, les mèches sont rincées, puis lavées avec du shampooing Elsève multivitamines. On mesure les paramètres L*a*b* des cheveux.

De la même manière, une composition oxydante A2 classique sous forme de crème du type Platinium 20 vol (= 12% H202) de même teneur en H2O2 que l'émulsion A1mais contenant 8 % de corps gras, est mélangée à la composition B dans un rapport 15/14. Le mélange est ensuite appliqué sur des cheveux naturels châtains (hauteur de ton = 4). Le rapport de bain « mélange/mèche » est respectivement de 10/1 (g/g). Le temps de pose est de 30min à 27°C. A l'issue de ce temps, les mèches sont rincées, puis lavées avec du shampooing Elsève multivitamines. On mesure les paramètres Lab des cheveux.

### Résultats :

On détermine ΔE l'écart de couleur entre la mèche non décolorée (mèche non traitée) et la mèche décolorée

| | L* | a* | b* | ΔE |
|---|---|---|---|---|
| Cheveu non traité | **17,62** | **2,12** | **1,95** | ----- |
| cheveu traité avec mélange (composition A2+ composition B) | **21,77** | **6,44** | **7,72** | 8,32 |
| Cheveu traité avec mélange (composition A 1+ composition B) | **23,95** | **7,36** | **9,55** | 11,19 |

On constate que la composition A1 décolore le cheveu de façon plus efficace que la composition A2.

## Revendications

1. Procédé d'éclaircissement de matières kératiniques qui consiste à traiter les matières kératiniques avec au moins :
a) une émulsion directe (A) huile dans eau comprenant un ou plusieurs corps gras différents des acides gras dont au moins une huile, la ou les corps gras étant présent en quantité supérieure à 25 % en poids, un ou plusieurs tensioactifs non ioniques oxyéthylénés (OE) choisis parmi les alcools oxyéthylénés en C₁₈-C₃₀, le nombre de motifs OE étant compris entre 1 et 50 ; un ou plusieurs agents oxydants et une quantité d'eau supérieure à 5 % en poids, du pois total de l'émulsion,
b) une composition (B) comprenant un ou plusieurs agents alcalins choisis parmi les amines organiques.

2. Procédé selon la revendication précédente, **caractérisé en ce que** l'émulsion (A) comprend plus de 50 % en poids de corps gras.

3. Procédé selon la revendication 1 ou 2 dans lequel la teneur en eau dans l'émulsion (A) est supérieure à 10 % en poids.

4. Procédé selon l'une quelconque des revendications précédentes, **caractérisé en ce que** le ou les corps gras sont choisis parmi les composés liquides ou pâteux et de préférence liquide à température ambiante et à pression atmosphérique.

5. Procédé selon l'une quelconque des revendications précédentes, **caractérisé en ce que** le ou les corps gras sont choisis parmi les alcanes en C6-C16, les alcools gras non oxyalkylénés, les esters d'acide gras, les esters d'alcool gras, les huiles non siliconées minérales à plus de 16 atomes de carbone, végétales, animales ou synthétiques, les silicones, les cires non siliconées.

6. Procédé selon l'une quelconque des revendications précédentes, **caractérisé en ce que** le ou les corps gras sont choisis parmi l'huile de vaseline, les polydécènes, les esters d'acides gras ou d'alcools gras, liquides ou leurs mélanges.

7. Procédé selon l'une quelconque des revendications précédentes, **caractérisé en ce que** le ou les corps gras liquides sont choisis parmi des huiles de poids moléculaire > 360 g/mol.

8. Procédé selon l'une quelconque des revendications précédentes, **caractérisé en ce que** la teneur en corps gras est comprise entre 25 et 80% en poids par rapport au poids de l'émulsion (A).

9. Procédé selon l'une quelconque des revendications précédentes, **caractérisé en ce que** l'émulsion (A) comprend un ou plusieurs tensioactifs non ioniques oxyéthylénés de HLB variant de 8 à 18.

10. Procédé selon la revendication précédente **caractérisé par le fait que** le tensioactif de l'émulsion A est le produit d'addition d'oxyde d'éthylène avec l'alcool béhénylique, notamment ceux comportant de 9 à 50 groupes oxyéthylénés, de préférence 10 groupes oxyéthylénés.

11. Procédé selon l'une quelconque des revendications précédentes, **caractérisé en ce que** le ou les agents oxydants sont choisis parmi le peroxyde d'hydrogène, le peroxyde d'urée, les bromates ou ferricyanures de métaux alcalins, les sels peroxygénés, les peracides et leurs précurseurs, et les percarbonates de métaux alcalins ou alcalino-terreux.

12. Procédé selon la revendication précédente, **caractérisé en ce que** l'agent alcalin est au moins une aminé organique alcanolamine, de préférence choisie parmi le 2- amino 2-méthyl 1-propanol, la monoéthanolamine ou leurs mélanges, un acide aminé basique choisi parmi l'arginine, l'histidine, la lysine, ou leurs mélanges.

13. Procédé selon l'une quelconque des revendications précédentes, **caractérisé en ce que** la composition (B) comprend un ou plusieurs colorants d'oxydation et/ou un ou plusieurs colorants directs.

14. Procédé selon l'une quelconque des revendications précédentes, **caractérisé en ce que** l'on applique sur les matières kératiniques, une composition obtenue par mélange extemporané, au moment de l'emploi, de l'émulsion (A) et de la composition (B).

15. Procédé selon l'une quelconque des revendications 1 à 13, **caractérisé en ce que** l'on applique sur les matières kératiniques, successivement et sans rinçage intermédiaire, la composition (B) et l'émulsion (A), l'ordre étant indifférent.

16. Procédé selon l'une quelconque des revendications précédentes, **caractérisé en ce que** les matières kératiniques sont des cheveux humains.

17. Dispositif à plusieurs compartiments comprenant dans un premier compartiment, l'émulsion (A) selon l'une des revendications 1 à 11 dans un autre compartiment une composition (B) telle que définie à l'une des revendications 1 et 12 à 14.

## Patentansprüche

1. Verfahren zur Aufhellung von Keratinsubstanzen, bei dem man die Keratinsubstanzen mit mindestens den Folgenden behandelt:
a) einer direkten Öl-in-Wasser-Emulsion (A), umfassend ein oder mehrere verschiedene Fette von Fettsäuren, einschließlich mindestens eines Öls, wobei das oder die Fett (e) in einer Menge von mehr als 25 Gew.-% vorliegt/vorliegen, ein oder mehrere oxyethylenierte (OE) nichtionische Tenside, ausgewählt aus oxyethylenierten C₁₈-C₃₀-Alkoholen, wobei die Anzahl an OE-Motiven zwischen 1 und 50 beträgt; ein oder mehrere Oxidationsmittel und eine Menge an Wasser von mehr als 5 Gew.-%, bezogen auf das Gesamtgewicht der Emulsion,
b) einer Zusammensetzung (B), die ein oder mehrere aus organischen Aminen ausgewählte alkalische Mittel umfasst.

2. Verfahren nach dem vorhergehenden Anspruch, **dadurch gekennzeichnet, dass** die Emulsion (A) mehr als 50 Gew.-% Fett enthält.

3. Verfahren nach Anspruch 1 oder 2, wobei der Gehalt an Wasser in der Emulsion (A) höher als 10 Gew.-% ist.

4. Verfahren nach einem der vorhergehenden Ansprüche, **dadurch gekennzeichnet, dass** das Fett oder die Fette aus Verbindungen ausgewählt ist/sind, die bei Umgebungstemperatur und Atmosphärendruck flüssig oder pastös und vorzugsweise flüssig sind.

5. Verfahren nach einem der vorhergehenden Ansprüche, **dadurch gekennzeichnet, dass** das Fett oder die Fette aus C₆-C₁₆-Alkanen, nicht-oxyalkylenierten Fettalkoholen, Fettsäureestern, Fettalkoholestern, nicht-silikonhaltigen Mineralölen mit mehr als 16 Kohlenstoffatomen, pflanzlichen, tierischen oder synthetischen Ölen, Silikonen, nicht-silikonhaltigen Wachsen ausgewählt ist/sind.

6. Verfahren nach einem der vorhergehenden Ansprüche, **dadurch gekennzeichnet, dass** das Fett oder die Fette aus Vaselinöl, Polydecenen, Fettsäure- oder Fettalkoholestern, die flüssig sind, oder deren Gemischen ausgewählt ist/sind.

7. Verfahren nach einem der vorhergehenden Ansprüche, **dadurch gekennzeichnet, dass** das oder die flüssige(n) Fett(e) aus Ölen mit einem Molekulargewicht > 360 g/mol ausgewählt ist/sind.

8. Verfahren nach einem der vorhergehenden Ansprüche, **dadurch gekennzeichnet, dass** der Gehalt an Fett zwischen 25 und 80 Gew.-%, bezogen auf das Gewicht der Emulsion (A), beträgt.

9. Verfahren nach einem der vorhergehenden Ansprüche, **dadurch gekennzeichnet, dass** die Emulsion (A) ein oder mehrere oxyethylenierte nichtionische Tenside mit einer HLB im Bereich von 8 bis 18 umfasst.

10. Verfahren nach dem vorhergehenden Anspruch, **dadurch gekennzeichnet, dass** das Tensid der Emulsion (A) das Produkt der Addition von Ethylenoxid an Behenylalkohol, insbesondere denjenigen, die 9 bis 50 Oxyethylengruppen, vorzugsweise 10 Oxyethylengruppen umfassen, ist.

11. Verfahren nach einem der vorhergehenden Ansprüche, **dadurch gekennzeichnet, dass** das oder die Oxidationsmittel aus Wasserstoffperoxid, Harnstoffperoxid, Alkalimetallbromaten oder -ferricyaniden, Peroxidsalzen, Persäuren und deren Vorstufen und Alkalimetall- oder Erdalkalimetallpercarbonaten ausgewählt ist/sind.

12. Verfahren nach dem vorhergehenden Anspruch, **dadurch gekennzeichnet, dass** das alkalische Mittel mindestens ein Alkanolamin mit organischer Amingruppe, vorzugsweise ausgewählt aus 2-Amino-2-methyl-1-propanol, Monoethanolamin oder deren Gemischen, eine basische Aminosäure, ausgewählt aus Arginin, Histidin, Lysin oder deren Gemischen, ist.

13. Verfahren nach einem der vorhergehenden Ansprüche, **dadurch gekennzeichnet, dass** die Zusammensetzung (B) einen oder mehrere Oxidationsfarbstoffe und/oder einen oder mehrere Direktfarbstoffe umfasst.

14. Verfahren nach einem der vorhergehenden Ansprüche, **dadurch gekennzeichnet, dass** man auf die Keratinsubstanzen eine Zusammensetzung aufbringt, die durch Mischen der Emulsion (A) und der Zusammensetzung (B) kurz vor Gebrauch im Moment der Verwendung erhalten wird.

15. Verfahren nach einem der Ansprüche 1 bis 13, **dadurch gekennzeichnet, dass** man auf die Keratinsubstanzen nacheinander und ohne Zwischenspülen die Zusammensetzung (B) und die Emulsion (A) in beliebiger Reihenfolge aufbringt.

16. Verfahren nach einem der vorhergehenden Ansprüche, **dadurch gekennzeichnet, dass** die Keratinsubstanzen menschliche Haare sind.

17. Vorrichtung mit mehreren Kompartimenten, die in einem ersten Kompartiment die Emulsion (A) nach einem der Ansprüche 1 bis 11, in einem anderen Kompartiment eine Zusammensetzung (B) nach einem der Ansprüche 1 und 12 bis 14 umfasst.

## Claims

1. Method for lightening keratin materials, which consists in treating the keratin materials with at least:
a) a direct oil-in-water emulsion (A) comprising one or more fatty substances other than fatty acids, including at least one oil, the fatty substance(s) being present in an amount greater than 25% by weight, one or more oxyethylenated (OE) non-ionic surfactants chosen from C₁₈-C₃₀ oxyethylenated alcohols, the number of OE units being between 1 and 50; one or more oxidizing agents and an amount of water greater than 5% by weight of the total weight of the emulsion,
b) a composition (B) comprising one or more alkaline agents chosen from the organic amines.

2. Method according to the preceding claim, **characterized in that** the emulsion (A) comprises more than 50% by weight of fatty substance.

3. Method according to Claim 1 or 2, wherein the water content in the emulsion (A) is greater than 10% by weight.

4. Method according to any one of the preceding claims, **characterized in that** the fatty substance(s) are chosen from liquid or pasty compounds and preferably compounds that are liquid at room temperature and atmospheric pressure.

5. Method according to any one of the preceding claims, **characterized in that** the fatty substance(s) are chosen from C6-C16 alkanes, non-oxyalkylenated fatty alcohols, fatty acid esters, fatty alcohol esters, non-silicone mineral oils containing more than 16 carbon atoms, plant, animal or synthetic oils, silicones, or non-silicone waxes.

6. Method according to any one of the preceding claims, **characterized in that** the fatty substance(s) are chosen from liquid petroleum jelly, polydecenes, esters of fatty acids or of fatty alcohols, which are liquid, or mixtures thereof.

7. Method according to any one of the preceding claims, **characterized in that** the liquid fatty substance(s) are chosen from oils of molecular weight >360 g/mol.

8. Method according to any one of the preceding claims, **characterized in that** the content of fatty substance is between 25 and 80% by weight relative to the weight of the emulsion (A).

9. Method according to any one of the preceding claims, **characterized in that** the emulsion (A) comprises one or more oxyethylenated non-ionic surfactants with HLB ranging from 8 to 18.

10. Method according to the preceding claim, **characterized in that** the surfactant of the emulsion A is the addition product of ethylene oxide with behenyl alcohol, especially those comprising from 9 to 50 oxyethylenated groups, preferably 10 oxyethylenated groups.

11. Method according to any one of the preceding claims, **characterized in that** the oxidizing agent(s) are chosen from hydrogen peroxide, urea peroxide, alkali metal bromates or ferricyanides, peroxygenated salts, peracids and precursors thereof, and alkali metal or alkaline earth metal percarbonates.

12. Method according to the preceding claim, **characterized in that** the alkaline agent is at least one alkanolamine organic amine, preferably chosen from 2-amino-2-methyl-1-propanol, monoethanolamine, or mixtures thereof, a basic amino acid chosen from arginine, histidine, lysine, or mixtures thereof.

13. Method according to any one of the preceding claims, **characterized in that** the composition (B) comprises one or more oxidation dyes and/or one or more direct dyes.

14. Method according to any one of the preceding claims, **characterized in that** a composition obtained by extemporaneous mixing, at the moment of use, of the emulsion (A) and of the composition (B) is applied to the keratin materials.

15. Method according to any one of Claims 1 to 13, **characterized in that** the composition (B) and the emulsion (A) are applied to the keratin materials successively and without intermediate rinsing, the order being unimportant.

16. Method according to any one of the preceding claims, **characterized in that** the keratin materials are human hair.

17. Multi-compartment device comprising, in a first compartment, the emulsion (A) according to one of Claims 1 to 11, and in another compartment a composition (B) as defined in one of Claims 1 and 12 to 14.
